# EUROPEAN PATENT SPECIFICATION

(11) **EP 1 495 329 B1**
(45) Date of publication and mention of the grant of the patent: **29.03.2006**
(21) Application number: 02722595.2
(22) Date of filing: 18.04.2002
(51) Int. Cl.: G01N 33/68, G01N 33/50, C12Q 1/68, C12Q 1/02

(54) **METHOD FOR THE IDENTIFICATION OF MODULATORS OF A SECRETASE ACTIVITY**
VERFAHREN ZUM NACHWEIS VON MODULATOREN EINER SEKRETASEAKTIVITÄT
PROCEDE POUR IDENTIFIER DES MODULATEURS D'UNE ACTIVITE DE SECRETASE

(43) Date of publication of application: 12.01.2005
(73) Proprietor: Esbatech AG, 8952 Schlieren (CH)
(72) Inventor: BARBERIS, Alcide, CH-8057 Zürich (CH); MIDDENDORP, Oliver, Michael, CH-8400 Winterthur (CH); LÜTHI, Urs, CH-8180 Bülach (CH)
(74) Representative: Blum, Rudolf Emil
(86) International application number: PCT/IB2002/001342
(87) International publication number: WO 2003/087842

(56) References cited:
- WO-A-00/34511
- WO-A-01/62897
- WO-A-01/75088
- WO-A-02/06306
- WO-A-98/13488
- DE-A- 19 920 514
- US-A1- 2002 025 508
- US-B1- 6 333 167
- KARLSTROM HELENA ET AL: "A sensitive and quantitative assay for measuring cleavage of presenilin substrates." JOURNAL OF BIOLOGICAL CHEMISTRY, vol. 277, no. 9, 1 March 2002 (2002-03-01), pages 6763-6766, XP002231028 March 1, 2002 ISSN: 0021-9258
- DIXON E P ET AL: "AN INVERSE MAMMALIAN TWO-HYBRID SYSTEM FOR BETA SECRETASE AND OTHERPROTEASES" ANALYTICAL BIOCHEMISTRY, ACADEMIC PRESS, SAN DIEGO, CA, US, vol. 249, no. 2, 1997, pages 239-241, XP000914815 ISSN: 0003-2697
- FEEHAN C ET AL: "SHEDDING OF THE LYMPHOCYTE L-SELECTIN ADHESION MOLECULE IS INHIBITED BY A HYDROXAMIC ACID-BASED PROTEASE INHIBITOR IDENTIFICATION WITH AN L-SELECTIN-ALKALINE PHOSPHATASE REPORTER" JOURNAL OF BIOLOGICAL CHEMISTRY, AMERICAN SOCIETY OF BIOLOGICAL CHEMISTS, BALTIMORE, MD, US, vol. 271, no. 12, 22 March 1996 (1996-03-22), pages 7019-7024, XP000616670 ISSN: 0021-9258

### SEQUENCE LISTING

<110> ESBATech AG
<120> Method for the identification of modulators of a secretase activity
<130> 05606PC
<160> 1
<170> PatentIn version 3.1
<210> 1
   <211> 6
   <212> PRT
   <213> Artificial sequence
<220>
   <223> ER retention peptide
<400> 1
   Asp Glu Lys Lys Met Pro
   1               5

## Description

### Technical Field

The present invention relates to a *in vivo* method for the identification of modulators of secretase activities.

### Background Art

Protein secretion is central to the proper development and function of eukaryotic organisms. Moreover, several pathophysiological processes such as neurodegeneration, oncogenesis, apoptosis and inflammation are associated with the malfunction or aberrant regulation of protein secretion. It has become clear that there is no single biosynthetic mechanism common to all secretory proteins. Secretion of proteins can occur through either the regulated or constitutive pathways and, in some cell types, this secretion can be polarized to distinct cellular domains. An increasing number of proteins are now recognized as being derived from integral membrane proteins of type I and type II topology and, in this case, the secretory event involves their selective post-translational hydrolysis from the cell surface. This secretion is catalyzed by proteases known as secretases. The cleavage of membrane proteins generally occurs near the extracellular face of the membrane, although in some cases it has been shown also to occur within the transmembrane domain. Proteins secreted in this fashion include membrane receptors and receptor ligands, ectoenzymes, cell adhesion molecules and others. Examples of protein secretion through the action of secretases include the vasoregulatory enzyme ACE (angiotensin converting enzyme), the tumor necrosis factor (TNF) ligand and receptor superfamilies, the transforming growth factor-α, certain cytokine receptors, the Alzheimer's amyloid precursor protein (APP) and others (7).

The involvement of secretases in the development of human diseases makes them potential drug target candidates in a variety of disease areas, including anticancer drugs, cardiovascular drugs, anti-neurodegenerative drugs and anti-inflammatory drugs.

In the past, different in vitro/in vivo and biochemical methods have been used to identify modulators of secretease enzymes.

US patent No. 5,942,400 and WO 96/40885 disclose *in vitro* methods for screening for candidate drugs for the ability to inhibit the activity of beta-secretase. Said methods are based on the detection of APP cleavage products using specific antibodies.

WO 98/13488 describes a method for determining the activity of modulators of APP secretases that are active in cultured human cells. Said method involves transfection of tissue-culture cells with vectors that express cleavable reporter proteins. Upon cleavage by the endogenous secretases, a reporter domain is secreted and can be detected using standard biochemical and immunological methods.

WO 01/49871 discloses an in vivo process for finding substances which specifically inhibit γ-secretase. Said process encompasses cells expressing a secretase activity and a fusion protein which comprises the substrate of said secretase with the specific cleavage site and a reporter. Said cells are contacted with a test substance and the quantity of reporter cleaved is either measured directly or indirectly. Suitable reporter proteins allowing direct detection are GFP, luciferase, β-galactosidase and secreted alkaline phosphatase. In case of an indirect detection of the reporter, the cleaved reporter is a transcription factor or part of a transcription factor which migrates into the nucleus and induces expression of a reporter e.g. luciferase.

WO01/75088 discloses a method for the identification of modulators of a secretase activity ("an interaction partner protein", page 5, line 5; or "a protein that is able to process a specific secretase"; page 6, line 33) in an eukaryotic host cell system (mutant yeast). Said cells express a transmembrane fusion protein comprising an invertase secretory fragment, a secretase cleavage sequence (a-secretase), and a transcriptional activator which is part of a reporter system stably integrated into the host cell genome (HIS3, for example) (see page 5, line 27, to page 6, line 24). The secretory fragment is a functional invertase protein (aa 1-532 of SUC2) which confers sensitivity to a chemical (sucrose, glucose, galactose), represents a second reporter gene complementing auxotrophy (SUC2), and has an enzymatic activity.

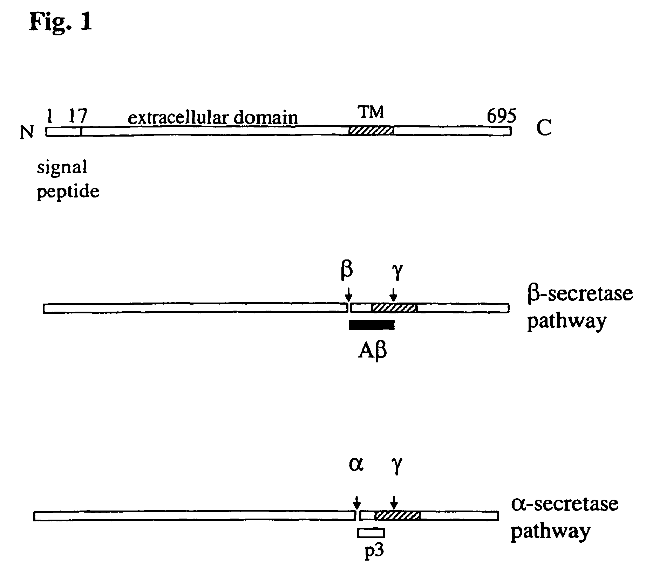

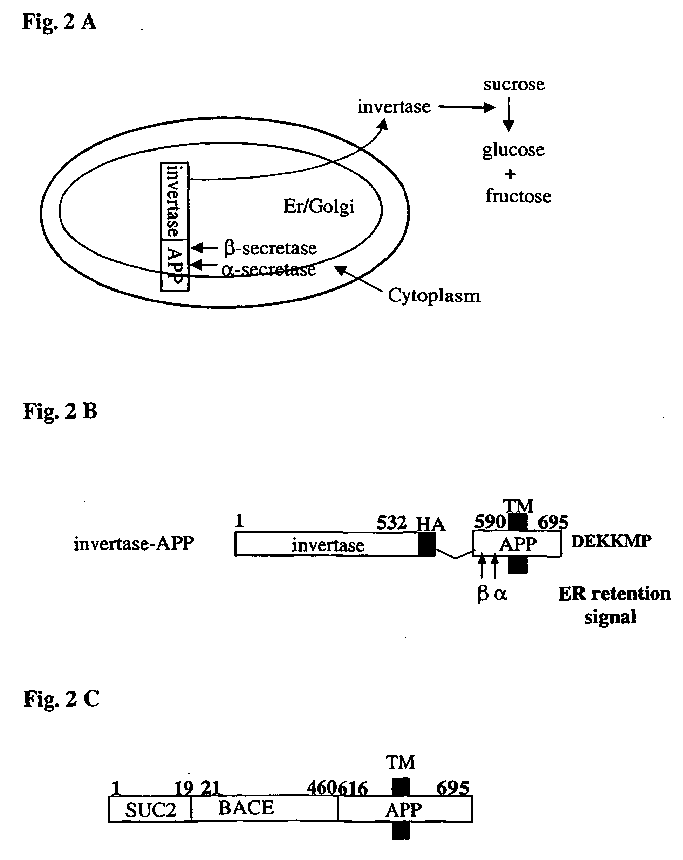

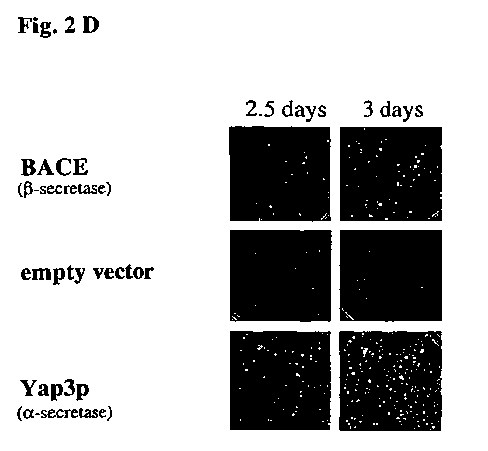

WO00/34511 discloses a method for the identification of modulators of a secretase activity in an eukaryotic cell system (see page 5, line 15, to page 8, line 8; page 11, line 23, to page 12, line 16; Figures 1 and 2). The substrate is a fusion protein comprising secretory fragment, membrane anchor and cleavage sequence (C100 fragment of APP fused to GAL4-VP16 activation domain). Upon γ-secretase cleavage, the GAL4-VP16 activation domain is translocated to the nucleus, where it activates a reporter gene (GFP) under the control of a GAL4 transcriptional activation system.

US2002/025508 describes a similar method to identify inhibitors of membrane-bound proteases (including secretases) with a fusion substrate comprising an APP fragment and a GAL4 fragment. The γ-secretase contains and ER retention signal. Upon cleavage, the GAL4 protein is released and translocated to the nucleus where it activates the transcription of the luciferase reporter system (see Abstract; pages 2-4; Figures 1 and 2; Example 3).

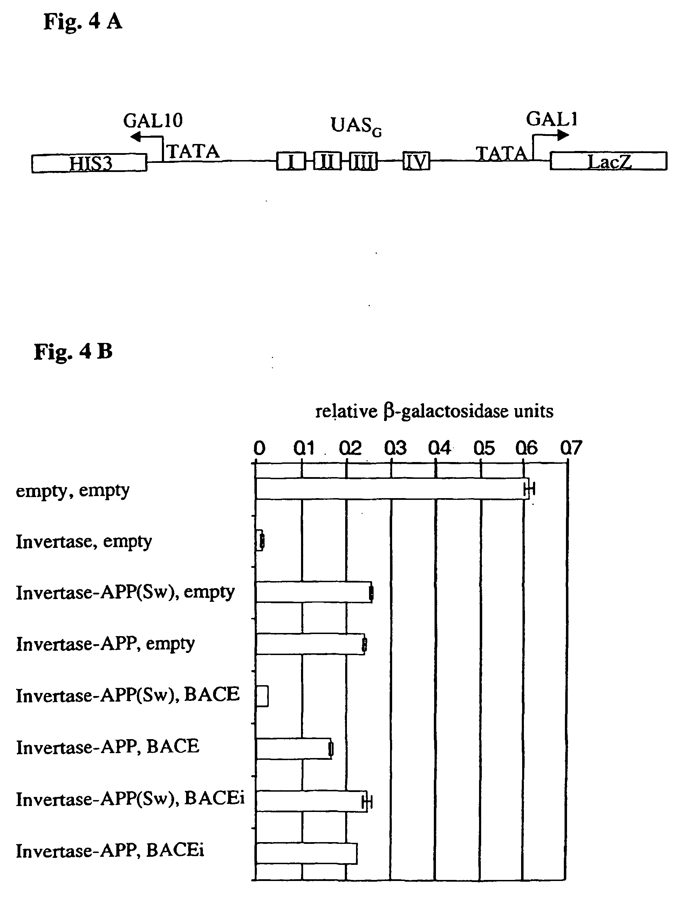

WO01/62897 describes a method to identify modulators of intramembrane proteolytic activities in an eukaryotic host cell system (see Abstract; Figure 4A; Example 5). An APP-Notch transmembrane chimeric protein is used as a substrate of γ-secretase; when the intracellular Notch fragment is released by proteolysis, it is translocated to the nucleus, activating a luciferase reporter system.

US-B1-6333167 discloses a method following the same principle described above, but using a different chimeric substrate: P450 extracellular and transmembrane fragment, intracellular lac I-NLS transcriptional repressor fragment, both fragments joined by a protease cleavage site string. Upon cleavage, the lac I-NLS fragment is released and translocated to the nucleus, where it represses the transcription of the RSVO-luciferase reporter system (see Abstract; Figures 1 and 2; column 3, line 5, to column 4, line 19; column 8, line 47, to column 10, line 2).

Although the prior art already discloses screening methods for secretase modulators, there is a need for improved reliable in vivo screening systems for the identification of modulators of a secretase activity.

### Disclosure of the Invention

Hence, it is a general object of the present invention to provide a method for the identification/isolation of modulators of a secretase activity. Said method is defined by the following steps:
suitable eukaryotic host cells that are contacted with a test substance wherein said suitable host cells comprise:
   a) a fusion protein comprising a secretory protein, a membrane anchor domain and at least one secretase cleavage sequence,
   b) a protein comprising a secretase activity recognising said cleavage sequence of said fusion protein and
   c) at least one reporter gene under control of a transcriptional activation system wherein said transcriptional activation system is regulated by the release of said secretory protein from said fusion protein and its subsequent secretion
   then culturing said cells under suitable conditions such that said reporter gene allowing detection and/or survival of cells is only expressed or repressed in a manner that is dependent on an altered secretase activity due to said test substance.

In a preferred embodiment, the present invention relates to a method for the identification of a secretase inhibitor. Said method is characterised in that a reduced or no release of said secretory protein from said fusion protein due to a reduced/inhibited secretase activity leads to a reduced or no secretion of said secretory protein and to the induction of expression of said reporter gene. The induction of expression of said reporter gene allows under suitable culturing conditions the detection and/or survival of cells which are in contact with a test compound having an inhibitory effect on the secretase activity.

Said reporter gene is preferably selected from genes conferring antibiotic resistance, genes complementing auxotrophies and genes encoding reporter molecules with an activity that can be detected by colorimetric or fluorescent methods such as genes selected from the group consisting of: lacZ, Luciferase gene, green fluorescence protein gene and chloramphenicol acetyl transferase gene.

In another preferred embodiment, the present invention relates to a method for the identification of stimulators of a secretase activity. Said method is characterised in that the release of said secretory protein from said fusion protein due to an enhanced secretase activity leads to repression of said reporter gene expression thereby allowing detection and/or survival of cells which are in contact with a test substance having an stimulating effect on the secretase activity. Appropriate culturing conditions of the cells must be used such that said fusion protein is not efficiently cleaved by the secretase in the absence of said test substance having a stimulating effect on the secretase activity.

Said reporter gene is preferably selected from genes such as CYH2 or CAN1 conferring sensitivity to a chemical.

In a further preferred embodiment of the invention said suitable host cells comprise a second reporter gene that is selected from the group consisting of:
a) genes encoding reporter molecules with an activity that can be detected by colorimetric or fluorescent methods such as genes selected from the group consisting of: lacZ, luciferase gene, green fluorescence gene (GFP) and chloramphenicol acetyl transferase gene (CAT),
b) genes conferring antibiotic resistance,
c) genes conferring sensitivity to a chemical and
d) genes complementing auxotrophies.
Illustrative examples of suitable reporter genes that can be used in the present invention are mentioned above.

In a method of the present invention any eukaryotic cell can be used, preferably a yeast cell.

The term membrane anchor domain as used herein refers to molecules and/or protein domains which are responsible for the membrane association of a protein and includes e.g. transmembrane domains and GPI anchors.

The term secretory protein as used herein encompasses polypeptides or fragments thereof which are destined for export. Said secretory protein has preferably an enzymatic activity, more preferably it is a protein with invertase activity or functional fragments of a protein with invertase activity. An especially preferred invertase is a yeast invertase or functional fragments thereof. Yet it is obvious for the man skilled in the art that other secretory proteins can be used in a method of the present invention.

Any recognition sequence of a known secretase can be used for the construction of said fusion protein of the present invention. Preferred secretase recognition sites are selected from the β site and the α site of the human amyloid precursor protein (APP) and the S2 site of Notch 1 protein. A much preferred recognition site is the β site of human APP in which the Lys595Asn and Met596Leu changes were introduced (Swedish APP mutant) .

In a much preferred embodiment of the invention said fusion protein comprises amino acid residues 1-532 of yeast invertase, amino acid residues 590-695 of human APP and optionally an ER retention signal.

In a further preferred embodiments said fusion protein comprises amino acid residues 1-532 of yeast invertase, amino acid residues 1714-1876 of human Notch 1 and optionally an ER retention signal.

Said fusion protein can be expressed from an extrachromosomal gene construct e.g. from an episomal vector enabling expression of the fusion protein in a host cell. Preferably the nucleic acid construct encoding the fusion protein is integrated into the genome of the host cell. The nucleic acid can be introduced into the cell by any transfection method leading to uptake of the nucleic acid sequence into the cell. Such methods are know to the man skilled in the art and are e.g described in Sambrook et al., Molecular Cloning: A Laboratory Manual, New York: Cold Spring Harbor Laboratory, 2001).

The term secretase activity as used herein encompasses proteolytic enzymes or functional fragments thereof which cleave membrane associated proteins, integral membrane proteins of type I and type II topology.

In a preferred embodiment said protein comprising a secretase activity further comprises an ER signal sequence.

Preferred secretase activities for the present invention are selected from β-secretase and α-secretase of human APP.

In a preferred embodiment of the invention said protein comprising a β-secretase activity further comprises an ER signal sequence and amino acid residues 616-695 of human APP.

The protein comprising said secretase activity can be expressed endogenously by the host cell or it can be encoded by a nucleic acid construct e.g. an episomal expression vector or by a nucleic acid construct that is stably integrated into the genome of the host cell.

### Brief Description of the Drawings

The invention will be better understood and objects other than those set forth above will become apparent when consideration is given to the following detailed description thereof. Such description makes reference to the annexed drawings, wherein:
Fig. 1 shows the primary structure and cellular processing of APP. APP is processed by a sequential cleavage reaction during trafficking through the secretory pathway. The α-secretase pathway is the preferred, constitutive pathway releasing, in combination with the γ-secretase, a non-toxic p3 fragment. In Alzheimer patients, the α-secretase pathway is competed by the amyloidogenic β-secretase pathway, where, upon γ-secretase cleavage, secretion of the Aβ-peptide leads to formation of extracellular plaques;
Fig. 2A shows the invertase selection system. A portion of APP harboring the α- and the β-site is fused to the enzyme invertase. The β-secretase (BACE) is co-expressed with the invertase-APP fusion protein. Cleavage of the invertase-APP reporter at the β-site liberates the invertase. Secretion of the invertase leads to cleavage of sucrose into glucose and fructose, thus enabling growth on sucrose plates;
Fig. 2B shows a schematic drawing of the invertase-APP reporter. The invertase-APP reporter was constructed by fusing the full-length invertase (aa 1-532) to a portion of APP (aa 590-695) harboring the secretase sites, the transmembrane domain and the cytoplasmic tail. Fusion of the ER retention signal DEKKMP (Seq. Id. No. 1) to the C-term further retards growth of clones that do not express an active secretase;
Fig. 2C shows a schematic drawing of the engineered BACE. The signal sequence of BACE was replaced by the SUC2 signal sequence (aa 1-19) to ensure efficient translocation into the yeast ER. To achieve colocalization of BACE with the invertase-APP fusion protein, the C-term of BACE was substituted by a portion of APP harboring the transmembrane domain and the cytosolic tail (aa 616-695);
Fig. 2D shows that BACE enhances growth on sucrose plates. The invertase-APP fusion protein harboring the Swedish mutation was co-expressed with the indicated constructs [BACE, empty vector or Yap3p (a yeast secretase that specifically cleaves APP at the α-site)] in a yeast strain deficient for the suc2 gene and the endogenous α-secretases mkc7 and yap3. Growth on sucrose plates was monitored after 2.5 days and 3 days, respectively;

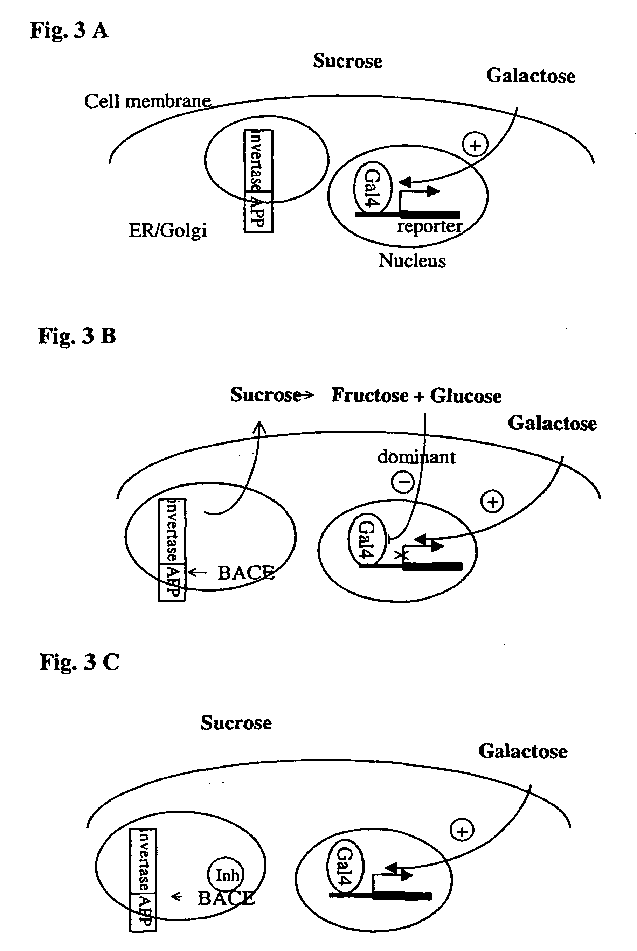

Fig. 3 shows principles of a cellular selection system to identify modulators of secretases. Fig. 3A-C show schematic drawings of yeast cells. The reporter gene is either the LacZ or different selectable marker genes, that can act either positively or negatively on growth. These genes are under the control of the Gall/10 promoter (denoted by the GAL4 transcription factor);
Fig. 3A shows default readout (without BACE): The reporter gene is induced. Biscrel cells harboring the invertase-APP fusion protein in the absence of any secretase activity express the reporter gene when grown on 5% sucrose/2% galactose. Galactose activates transcription of the reporter gene, whereas sucrose is inert to the system in the absence of secreted invertase;
Fig. 3B shows that in the presence of an active BACE the reporter gene is repressed. Cleavage of the invertase-APP(Sw) by the β-secretase leads to liberation and subsequent secretion of invertase. In the periplasm, invertase hydrolyses sucrose into glucose and fructose. The yielded glucose is taken up by the cell where it dominantly represses transcription of the reporter system;
Fig.3C shows that in the presence of an inhibited BACE the reporter gene is induced. If the action of BACE is blocked by a potent inhibitor the readout of the system is the same as in the absence of BACE (described in 3A);
Fig. 4A shows a schematic representation of the reporter gene. The HIS3 open reading frame (ORF) was fused to the 47 N-terminal amino acids of GAL10. The expression of the GAL10-HIS3 fusion protein is under the control of the GAL10 promoter, the expression of the LacZ ORF is controlled by the GAL1 promoter. The arrows indicate the transcription start points. The boxes labeled I, II, III and IV represent GAL4 binding sites;
Fig. 4B shows LacZ assays. Biscrel cells harboring the indicated constructs were cultivated in 5% sucrose 2% galactose -ura -trp drop out medium. Expression of the LacZ reporter gene was quantified using the β-galactosidase substrate ONPG. The columns represent the average β-galactosidase units of three samples each. Error bars are indicated. Invertase: soluble and secreted invertase; Invertase-APP: displayed in figure 2B; Invertase-APP(Sw): the same as invertase-APP, but harboring the Swedish mutation at the β-site; BACE: displayed in figure 2C; BACEi: the same as BACE, but harboring a point mutation in the active center, which renders the enzyme inactive. The constructs named "empty" only carry the yeast marker genes necessary to grow in the drop out medium,

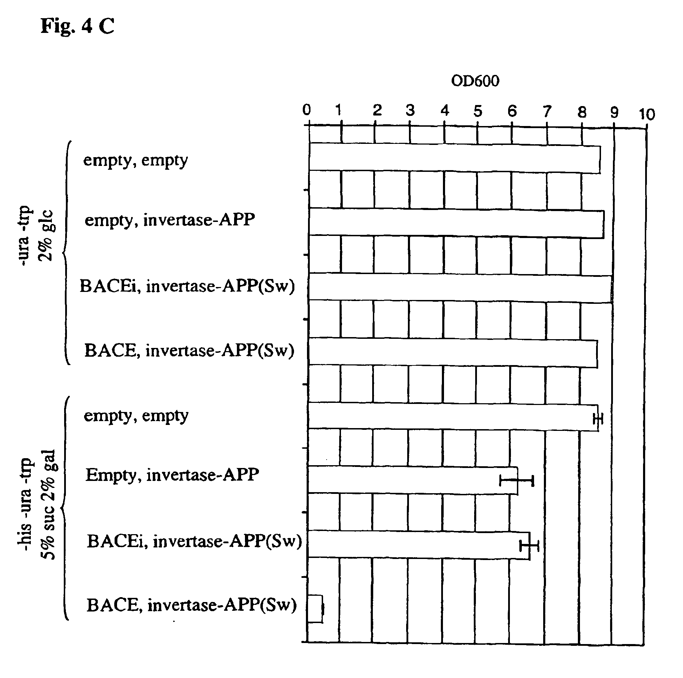

Fig. 4C shows liquid growth assays. Biscre1 cells harboring the indicated constructs were cultivated in non-selective -ura -trp 2% glucose medium as well as in selective -his -ura -trp 5% sucrose 2% galactose medium. After 24 h, the cell density of the cultures was determined by measuring the optical density (OD600). The cell density in the selective medium is an indicator for the expression of the HIS3 gene. For the explanation of the different constructs, see legend of figure 4B. For the selective cultures, the average OD600 of three cultures is displayed. The standard deviation is indicated by an error bar.

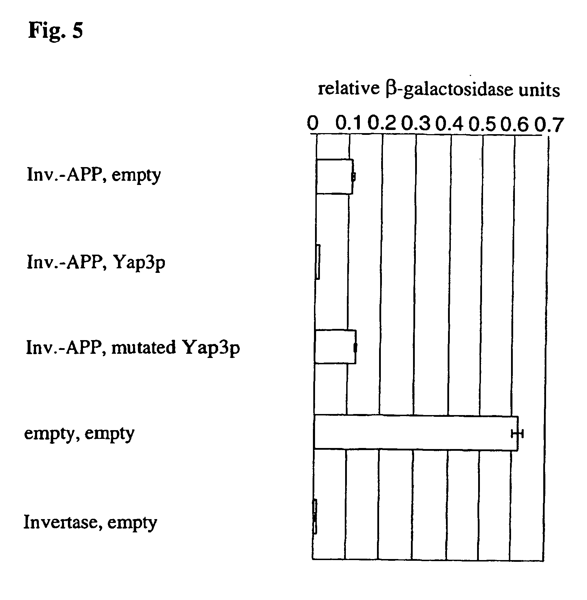

Fig. 5 shows that cleavage of invertase-APP by the yeast secretase Yap3p can be monitored by the cellular system to identify modulators of secretases. Induction of reporter gene expression (LacZ) was measured in a LacZ assay.

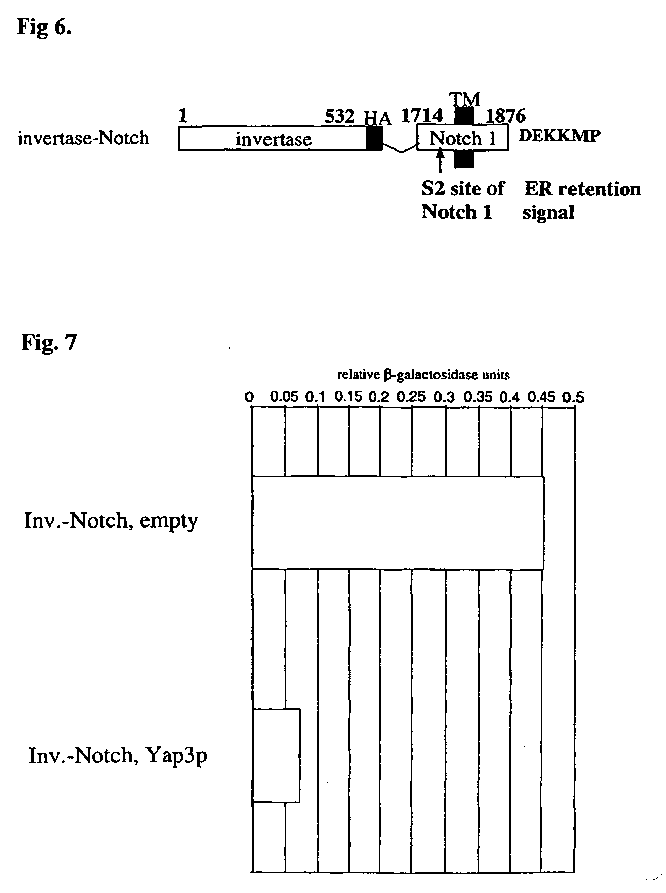

Fig. 6 shows a schematic drawing of the invertase-Notch 1 reporter. The invertase-Notch 1 reporter was constructed by fusing the full-length invertase (aa 1-532) to a portion of Notch 1 (aa 1714-1876) harboring the S2 site of Notch 1 protein, the transmembrane domain and the cytoplasmic tail. Fusion of the ER retention signal DEKKMP (Seq. Id. No. 1) to the C-term further retards growth of clones that do not express an active secretase and
Fig. 7 shows that cleavage of invertase-Notch by the yeast α-secretase Yap3p can be monitored by the cellular system to identify modulators of secretases. Induction of reporter gene expression (LacZ) was measured in a LacZ assay.

### Modes for Carrying Out the Invention

A reporter gene for the use in the present invention is under control of a transcriptional activation system. A preferred transcriptional activation system is a GAL gene regulatory system, more preferably a yeast GAL gene regulatory system.

In a preferred embodiment of the invention said reporter gene is under control of the yeast GAL1-10 gene regulatory region.

The use of said system in a preferred embodiment of the present invention has the following theoretical background:

The preferred carbohydrate of yeast cells is glucose since it can be metabolized directly. Other carbohydrates are converted to the glycolytic substrate glucose-1-phosphate in several steps. Addition of galactose to yeast cultures grown on glycerol induces expression of the GAL genes at least 1000-fold. If glucose is added to the galactose-containing media, the GAL genes are induced to only 1% of the levels obtained with galactose alone. This phenomenon is known as glucose repression. GAL2, GAL1-GAL7-GAL10, and MEL1 are the GAL structural genes. Their products transport galactose into the cell and convert it to glucose-1-phosphate. The major regulatory proteins of this system are the products of the genes GAL3, GAL4, and GAL80. Gal4p, the product of the GAL4 gene, is a transcriptional activator that binds specific regulatory DNA elements called UAS_{G}, which are present near the promoter regions of the galactose inducible genes. Its activity is required to stimulate expression of the GAL genes in the presence of galactose. Ga180p is a direct repressor of Ga14p, while Ga13p mediates the galactose-dependent release of Ga180p inhibition of Ga14p (for review see [5].

The glucose repression is exerted through several mechanisms [5]. Here, we focus on the regulation of the divergently oriented GAL1-10 genes, whose regulatory region was used for the experiments described below. The GAL1-10 regulatory region contains four UAS_{G} elements located between the divergent GAL1 and GAL10 promoters. These UAS_{G} elements are bound by Ga14p in a cooperative manner. Due to this cooperativity, the system is rather sensitive towards changes in the concentration of Ga14p, which is reduced 3- to 5-fold in the presence of glucose. Ga180p significantly contributes to glucose repression of the GAL1-10 genes by binding to Ga14p to mask its activation domain, thereby preventing expression of these GAL genes [6]. The GAL1 promoter contains an additional regulatory element, which is the binding site for the repressor Miglp. The activity of this repressor is also regulated by glucose. (A Miglp site is also present in the GAL4 promoter.)

The construction of suitable host cells and the other molecular biological reagents for the use in the present invention e.g. fusion protein constructs can be done using standard molecular biology techniques as described e.g. in Sambrook et al., Molecular Cloning: A Laboratory Manual, New York: Cold Spring Harbor Laboratory, 2001).

The man skilled in the art is as well able to determine suitable culturing conditions allowing the detection and/or survival of the used cells. Said conditions are dependent on the used genetic constructs and the host cells.

There are at least three different categories of compounds that can be screened by a screening method of the present invention: chemical libraries, natural product libraries and combinatorial libraries. Chemical libraries consist of structural analogues of known compounds. Natural product libraries are collections of microorganism, animals, plants or marine organisms which are used to create mixtures for screening by for example fermentation and extraction of broths from soil, plant or marine microorganisms or extraction of plants or marine organisms. Combinatorial libraries are composed of large numbers of peptides, oligonucleotides or organic compounds as a mixture. They are relatively easy to prepare e.g. by traditional synthesis methods, PCR or cloning.

In a screening test of the present invention a test compound can e.g. be added to the culture medium of the host cells or it can be expressed by the host cells e.g. from an expression construct. The expression of test substances within the host cells is e.g. suitable for the screening of peptide libraries.

The present invention is now further illustrated by means of examples.

### Reconstitution of secretase activities in yeast

To monitor BACE activity within yeast cells, we made use of the invertase reporter system described in our patent application WO 01/75088, Title: "Method for identification of polypeptides with protease activity" .

Secretion of invertase enables yeast to use sucrose as a carbon source. The invertase hydrolyses sucrose to yield fructose and glucose. For the reporter system described in our patent application WO 01/75088, the endogenous gene encoding the invertase (the SUC2 gene) was knocked out. A recombinant invertase was fused to the N-terminus of a portion of APP harboring the transmembrane domain as well as the α- and β-sites (residues 590-695). In addition, an ER retention signal was added to the C-terminus of this fusion construct (Fig. 2A, B). These modifications prevent invertase from being secreted and, consequently, the yeast cells are unable to efficiently grow on plates containing sucrose as the only carbohydrate. The system described here is based on the observation that, upon cleavage of the APP portion at the α- or β-site, the invertase is liberated and secreted in the extracellular environment, where it hydrolyzes sucrose into fructose and glucose.

There are two yeast endogenous secretases described in the literature which can cleave APP at the α-site: Yap3p and Mkc7 [1, 2]. Since these proteins have α-secretase activity that constitutively cleaves APP, their respective genes had to be knocked out in order to investigate β-secretase activity in yeast using the invertase reporter system described in WO 01/75088.

To detect APP-specific BACE activity within yeast cells, the protein had to be modified. Since transmembrane and cytosolic sequences contribute to the subcellular localization of transmembrane proteins, the transmembrane and cytosolic portion of BACE was substituted by the transmembrane and cytosolic portion of APP. In this way, co-localization of the enzyme with its substrate was facilitated. To ensure efficient translocation into the yeast ER, the signal sequence of BACE was substituted with the SUC2-signal peptide at the N-terminus of the protein (Fig. 2C). Since BACE cleaves the APP wild type less efficiently than the so-called Swedish APP mutant [3], the β-site of the invertase-APP (590-695) reporter construct was altered such as to introduce the Lys595Asn and Met596Leu changes indicated as invertase-APP (Sw). Figure 2D shows the growth effect on sucrose plates due to cleavage of the invertase-APP (Sw) reporter protein by BACE or Yap3p.

### Cellular system to screen for secretase inhibitors

In order to establish the described cellular system to screen for BACE inhibitors, we combined the BACE-dependent invertase secretion system with the endogenous GAL gene regulatory network.

A reporter construct was cloned which expresses the LacZ gene under the control of the GAL1 promoter and the divergently oriented HIS3 gene under the control of the GAL10 promoter. As in the case of the endogenous GAL1 and GAL10 genes, four UAS_{G} elements are located between these two promoters. This reporter construct was integrated in yeast cells deficient for suc2, mkc7 and yap3 to create the strain Biscrel. If this strain is grown in the presence of galactose, expression of the LacZ gene and the HIS3 gene is induced by the UAS_{G}-binding Ga14p activator. As in the case of the endogenous GAL1 and GAL10 genes, these reporter genes become dominantly repressed upon addition of glucose to the medium. The product of the HIS3 gene is needed for growth on histidine-depleted (-his) medium. Consequently, Biscrel can only grow on -his media that contain galactose but no glucose. The HIS3 reporter gene of the strain Biscrel, which lacks the endogenous invertase activity, is also induced by Ga14p when these cells grow in the presence of sucrose together with galactose (Fig 3A). However, if Biscrel is allowed to secrete the invertase, glucose is generated by the hydrolysis of sucrose. As consequences, the newly generated glucose dominantly represses the HIS3 gene expressed from the GAL10 promoter and growth on -his medium is inhibited.

Biscrel cultured on sucrose and galactose and expressing the invertase-APP (Sw) fusion protein provides a tool to select for inhibited BACE. Indeed, if BACE expressed in Biscrel is active, the membrane-anchored invertase-APP (Sw) fusion protein is cleaved at the β site and the invertase moiety is secreted. The secreted invertase hydrolyses sucrose into fructose and glucose, the latter of which represses the HIS3 and the LacZ reporter genes (Fig 3B). If BACE is inhibited, either by mutations or by an inhibiting compound, the invertase moiety remains anchored to the ER-membrane via the APP domain, the sucrose in the medium is not hydrolyzed, and expression of the HIS3 and LacZ genes is induced by galactose (Fig 3C).

### Results

Expression of the product of the LacZ gene, a β-galactosidase, can be measured by using the substrate analogue o-nitro-phenyl-β-galactopyranosid (ONPG). A cleavage product of ONPG has a yellow colour. OD measurement at 420 nm allows quantification of the reaction catalyzed by β-galactosidase, and indirectly of the LacZ-expression. This assay allowed us to investigate the effect of invertase-APP cleavage by BACE in the context of Biscrel.

Biscrel was transformed with plasmids expressing different β-secretase activities together with the invertase-APP (Sw) construct, or its wildtype variant (invertase-APP). Empty plasmids and an invertase-expressing plasmid were used as negative and positive controls, respectively. Liquid cultures were grown in 5 % sucrose, 2 % galactose, 0.1 % glucose drop-out medium (The medium contained histidine for this assay as the cultures should grow equally). The maximal level of LacZ expression in this assay was obtained with transformants harboring two empty plasmids. In these cells there was no invertase activity, and consequently no glucose was produced in the presence of sucrose. In this way, values of >0.6 β-galactosidase units were measured (Fig. 4B, column 1). Expression of soluble and secreted invertase resulted in a strong repression of the reporter gene (Fig. 4B, column 2). Expression of the invertase-APP (Sw) fusion protein alone resulted in a β-galactosidase activity of 0.25 units (Fig. 4B, column 3). Expression of the invertase-APP wild-type protein resulted in a comparable level of LacZ gene activation (Fig. 4B, column 4). The 2-3-fold reduction in LacZ expression observed in the presence of these invertase fusion proteins is most likely due to the residual appearance of the invertase moiety at the surface of the cell. This could be due to a small portion of the fusion protein that reaches the cell surface, or to some residual endogenous secretase activity present in the cell despite the fact that the two major endogenous α-secretases Yap3p and Mkc7p were knocked out.

Co-expression of BACE with the invertase-APP (Sw) fusion protein led to a significant reduction of β-galactosidase activity (0.026 β-galactosidase units, Fig. 4B, column 5), whereas co-expression with the construct harboring the APP wild-type sequence (invertase-APP) resulted in an only slight reduction (Fig. 4B, column 6). A catalytically inactive version of BACE (BACEi) behaved similarly as an empty plasmid when expressed together with either one of these fusion constructs (Fig. 4B, columns 7, 8).

These results show that the expression level of the reporter gene system is dependent on the presence of free invertase. Free invertase was generated either by direct expression of the natural SUC2 gene or by liberation from a membrane-bound fusion protein. The liberation and subsequent secretion of the invertase moiety only took place efficiently when the active BACE secretase was co-expressed with the invertase fusion protein harboring the Swedish mutation, thus underlying the specificity of the system.

The expression of the HIS3 gene was quantified by a growth assay in liquid medium. For this assay, Biscrel cells were cultivated in liquid -his medium containing 5% sucrose and 2% galactose (selective conditions). Two-ml cultures were inoculated with equal amounts of cells transformed with the constructs of interest. Cell density was measured after 24 h. The same amounts of cells were used to inoculate cultures in non-selective medium. This control experiment showed that none of the constructs per se had an effect on cell growth under non-selective conditions (Fig. 4C, columns 1-4). The cell densities measured after growth in selective medium are displayed in figure 4C, columns 5-8. Biscrel harboring two empty plasmids fully induced the HIS3 gene and could grow under selective conditions (Fig. 4C, column 5). The cell density of this culture was comparable to those of all the cultures grown in non-selective medium. The cells transformed with the invertase-APP (Sw) construct together with an empty plasmid or with the inactivated BACE grew to a density of about 75% of the one reached with two empty plasmids (Fig. 4C, column 6, 7). Expression of active BACE together with the invertase-APP (Sw) fusion protein reduced cell growth to about 10% of the values obtained with inactivated BACE (Fig. 4C, column 8). Therefore, this assay provides a tool to screen compound libraries to identify BACE inhibitors that are active in a eukaryotic cellular environment and that do not negatively affect basic cellular functions.

The screening system is as well suitable to screen for modulators of other secretases. Figure 5 shows the results of such an experiment. Yap3p is a yeast secretase which cleaves APP at the α-site. The experimental procedures were the same as described for fig. 4B. The mutated Yap3p as well as the wildtype version were provided on a yeast expression vector. Yap3p cleaves the invertase-APP fusion protein at the α-site thereby liberating the invertase. The observed effect on LacZ expression is comparable to the effect observed when soluble and secreted invertase was expressed (last column).

To further investigate the general applicability of our system, a different target, namely the human Notch 1 protein, was fused to invertase (Fig. 6). The features of this new construct are identical to those of the invertase-APP fusion protein, except that the APP portion is replaced by the amino acids 1714-1876 of the Notch 1 protein (NCBI Protein Databank accession number AAG33848), which include the transmembrane domain. The invertase-Notch construct was co-expressed with Yap3p (α-secretase) or with an empty vector in the Biscrel strain and a β-gal assay was performed. The results of this experiment are displayed in figure 7. The first column indicates the value obtained with the empty vector (0.4532 β-gal units); the second column indicates the value obtained for Yap3p (0.0724 β-gal units). The cleavage of the invertase-Notch fusion protein was confirmed by detecting the cleavage products in a Western blot (data not shown).

While there are shown and described presently preferred embodiments of the invention, it is to be distinctly understood that the invention is not limited thereto but may be otherwise variously embodied and practiced within the scope of the following claims.

### References

1. Zhang, W., et al., *Characterization of beta-amyloid peptide precursor processing by the yeast Yap3 and Mkc7 proteases.* Biochimica et Biophysica Acta, 1997. **1359**(2): p. 110-22.
2. Komano, H., et al., *Involvement of cell surface glycosyl-phosphatidylinositol-linked aspartyl proteases in alpha-secretase-type cleavage and ectodomain solubilization of human Alzheimer beta-amyloid precursor protein in yeast.* J Biol Chem, 1998. **273**(48): p. 31648-51.
3. Citron, M., et al., *Mutation of the beta-amyloid precursor protein in familial Alzheimer's disease increases beta-protein production.* Nature, 1992. **360**(6405): p. 672-4.
4. Lohr, D., P. Venkov, and J. Zlatanova, *Transcriptional regulation in the yeast GAL gene family: a complex genetic network.* The FASEB Journal, 1995. **9**: p. 777-786.
5. Lamphier, M.S. and M. Ptashne, *Multiple mechanisms mediate glucose repression of the yeast GAL1 gene.* Proc. Natl. Acad. Sci. USA, 1992. **89**: p. 5922-5926.
6. Melcher, K. and H.E. Xu, *Ga180-Ga180 interaction on adjacent Gal4p binding* sites *is required for complete GAL gene repression.* Embo J, 2001. **20**(4): p. 841-851.
7. Hooper, N.M., Karran, E.H., Turner, A.J. *Membrane protein secretases* Biochem. J, 1997. **321**: 265-279.

## Claims

1. A method for the identification/isolation of modulators of a secretase activity wherein
suitable eukaryotic host cells are contacted with a test substance wherein said suitable host cells comprise:
a) a fusion protein comprising a secretory protein, a membrane anchor domain and a secretase cleavage sequence,
b) a protein comprising a secretase activity recognising said cleavage sequence of said fusion protein and
c) at least one reporter gene under control of a transcriptional activation system wherein said transcriptional activation system is regulated by the release of said secretory protein from said fusion protein by said secretase activity and its subsequent secretion
then culturing said cells under suitable conditions such that said reporter gene allowing detection and/or survival of cells is only expressed or repressed in a manner that is dependent on an altered secretase activity due to said test substance.

2. Method for the isolation of a secretase inhibitor according to claim 1, wherein a reduced or no release of said secretory protein due to a reduced/ inhibited secretase activity leads to expression of said at least one reporter gene thereby allowing detection and/or survival of cells under suitable culturing conditions.

3. Method according to claim 1 or 2, wherein said at least one reporter gene is selected from genes conferring antibiotic resistance, genes encoding reporter molecules with an activity that can be detected by colorimetric or fluorescent methods and genes complementing auxotrophies, preferably a His3 gene.

4. Method for the identification of agonists of a secretase activity according to claim 1, wherein the release of said secretory protein due to an enhanced secretase activity leads to a reduced expression of said at least one reporter gene thereby allowing detection and/or survival of cells under suitable conditions.

5. Method according to claim 4, wherein said at least one reporter gene is selected from genes conferring sensitivity to a chemical.

6. Method according to anyone of the preceding claims, wherein said cells comprise a second reporter gene selected from the group consisting of:
a) genes encoding reporter molecules with an activity that can be detected by colorimetric or fluorescent methods,
b) genes conferring antibiotic resistance and genes conferring sensitivity to a chemical and
c) genes complementing auxotrophies.

7. Method according to anyone of the preceding claims, wherein said cell is a yeast cell.

8. Method according to anyone of the preceding claims, wherein said secretory protein has an enzymatic activity, preferably a protein with invertase activity or functional fragments of a protein with invertase activity.

9. Method according to anyone of the preceding claims, wherein said secretory protein is a yeast invertase or functional fragments of a yeast invertase.

10. Method according to anyone of the preceding claims, wherein said secretase cleavage sequence is selected from the β site and the α site of the human amyloid precursor protein and the S2 site of Notch 1 protein.

11. Method according to claim 10, wherein into said β site the Lys595Asn and Met596Leu changes were introduced.

12. Method according to anyone of the preceding claims, wherein said fusion protein comprises an ER retention signal.

13. Method according to anyone of the preceding claims, wherein said fusion protein comprises amino acid residues 1-532 of yeast invertase, amino acid residues 590-695 of human APP and an ER retention signal.

14. Method according to anyone of the preceding claims, wherein said fusion protein comprises amino acid residues 1-532 of yeast invertase, amino acid residues 1714-1876 of human Notch 1 and an ER retention signal.

15. Method according to anyone of the preceding claims, wherein a nucleic acid construct encoding said fusion protein is integrated into the genome of said host cell.

16. Method according to anyone of the preceding claims, wherein said protein comprising a secretase activity recognising said cleavage sequence of said fusion protein further comprises an ER signal sequence.

17. Method according to anyone of the preceding claims, wherein said secretase activity is selected from β-secretase and α-secretase.

18. Method according to claim 16, wherein said protein comprising a β-secretase activity further comprises an ER signal sequence and amino acid residues 616-695 of human APP.

19. Method according to anyone of the preceding claims, wherein said transcriptional activation system comprises at least part of the yeast GAL gene regulatory system.

20. Method according to anyone of the preceding claims, wherein said reporter gene is under control of the yeast GAL1-10 regulatory region.

## Patentansprüche

1. Ein Verfahren zur Identifiziertung/Isolierung von Modulatoren einer Sekretaseaktivität wobei
geeignete eukaryontische Wirtszellen mit einer Testsubstanz kontaktiert werden, wobei diese Wirtszellen enthalten:
a) ein Fusionsprotein umfassend ein ein sekretorisches Protein, eine Membranankerdomäne und eine Sekretaseschnittstelle,
b) ein Protein mit einer Sekretaseaktivität, welche die Schnittstelle des Fusionsproteins erkennt und
c) mindestens ein Reporter-Gen unter der Kontrolle eines transkriptionellen Aktivierungssystems, wobei dieses transkriptionelle Aktivierungssystem **dadurch** reguliert wird, dass das sekretorische Protein durch die Sekretaseaktivität von dem Fusionsprotein abgetrennt und anschliessend ausgeschieden wird,
diese Zellen dann unter geeigneten Bedingungen gezüchtet werden, so dass jenes Reporter-Gen, welches die Detektion und/oder das Überleben der Zellen gestattet, nur in einer Weise exprimiert oder reprimiert wird, die von einer durch die Testsubstanz veränderten Sekretaseaktivität abhängig ist.

2. Verfahren zur Isolierung eines Sekretaseinhibitors gemäss Anspruch 1, wobei eine verminderte oder keine Abspaltung des sekretorischen Proteins aufgrund von einer reduzierten/gehemmten Sekretaseaktivität zur Expression jenes zumindest einen Reporter-Gens führt, wodurch die Detektion und/oder das Überleben von Zellen unter geeigneten Kulturbedingungen gestattet wird.

3. Verfahren gemäss Anspruch 1 oder 2, wobei jenes zumindest eine Reporter-Gen ausgewählt ist aus Genen, die Antibiotikaresistenz verleihen, Genen die für Reportermoleküle codieren, welche eine mit colorimetrischen oder Fluoreszenz-Verfahren detektierbare Aktivität haben, und Genen, die Auxotrophien komplementieren, vorzugsweise ein His3-Gen.

4. Verfahren zur Identifizierung von Agonisten einer Sekretaseaktivität gemäss Anspruch 1, wobei das Abtrennen des sekretorischen Proteins aufgrund einer erhöhten Sekretaseaktivität zu einer verminderten Expression jenes zumindest einen Reportergens führt, wodurch die Detektion und/oder das Überleben der Zellen unter geeigneten Bedingungen gestattet wird.

5. Verfahren gemäss Anspruch 4, wobei dieses zumindest eine Reporter-Gen ausgewählt ist aus Genen, die Sensitivität gegenüber einer Chemikalie verleihen.

6. Verfahren gemäss irgend einem der vorhergehenden Ansprüche, wobei die Zellen ein zweites Reporter-Gen enthalten, das ausgewählt ist aus der Gruppe von
a) Genen, die für Reportermoleküle kodieren mit einer durch colorimetrische oder Fluoreszenz-Verfahren detektierbaren Aktivität,
b) Genen, die Antibiotikaresistenz verleihen und Genen, die Sensitivität gegenüber einer Chemikalie verleihen und
c) Genen, die Auxotrophien komplementieren.

7. Verfahren gemäss irgend einem der vorangehenden Ansprüche, wobei die besagte Zelle eine Hefezelle ist.

8. Verfahren gemäss irgend einem der vorangehenden Ansprüche, wobei das sekretorische Protein eine enzymatische Aktivität hat, vorzugsweise ein Protein mit Invertaseaktivität oder funktionelle Fragmente eines Proteins mit Invertaseaktivität.

9. Verfahren gemäss irgend einem der vorangehenden Ansprüche, wobei das sekretorische Protein eine Hefeinvertase ist oder funktionelle Fragmente einer Hefeinvertase.

10. Verfahren gemäss irgend einem der vorangehenden Ansprüche, wobei die Sekretaseschnittstelle aus der β-Stelle und der α-Stelle des humanen Amyloid-Vorläuferproteins und der S2-Stelle des Notch 1-Proteins ausgewählt ist.

11. Verfahren gemäss Anspruch 10, wobei in die β-Stelle die Lys595Asn und Met596Leu Änderungen eingeführt wurden.

12. Verfahren gemäss irgend einem der vorangehenden Ansprüche, wobei das Fusionsprotein ein ER-Retentionssignal umfasst.

13. Verfahren gemäss irgend einem der vorangehenden Ansprüche, wobei das Fusionsprotein die Aminosäurenreste 1-532 der Hefeinvertase, die Aminosäurenreste 590-695 des humanen APP und ein ER-Retentionssignal umfasst.

14. Verfahren gemäss irgend einem der vorangehenden Ansprüche, wobei das Fusionsprotein die Aminosäuren 1-532 der Hefeinvertase, die Aminosäurenreste 1714-1876 des humanen Notch 1 und ein ER-Retentionssignal umfasst.

15. Verfahren gemäss irgend einem der vorangehenden Ansprüche, wobei ein Nukleinsäurenkonstrukt, das für jenes Fusionsprotein codiert, in das Genom der Wirtszellen integriert ist.

16. Verfahren gemäss irgend einem der vorangehenden Ansprüche, wobei das Protein, welches eine Sekretaseaktivität umfasst, welche die Schnittstelle des Fusionsproteins erkennt, weiter ein ER-Retentionssignal umfasst.

17. Verfahren gemäss irgend einem der vorangehenden Ansprüche, wobei die Sekretaseaktivität ausgewählt wird aus β-Sekretase und α-Sekretase.

18. Verfahren gemäss Anspruch 16, wobei das Protein, welches eine β-Sekretaseaktivität umfasst, eine ER-Signalsequenz und die Aminosäurenreste 616-695 des humanen APP umfasst.

19. Verfahren gemäss irgend einem der vorangehenden Ansprüche, wobei das transkriptionelle Aktivierungssystem mindestens einen Teil des Hefe-GAL-Gen-Regulationssystems umfasst.

20. Verfahren gemäss irgend einem der vorangehenden Ansprüche, wobei das Reporter-Gen unter der Kontrolle der Hefe-GAL1-10-Regulationsregion ist.

## Revendications

1. Méthode pour l'identification/isolation de modulateurs d'activité de sécrétases dans laquelle
des cellules hôtes eucaryotes appropriées sont en contact avec une substance test et dans laquelle lesdites cellules hôtes appropriées comportent :
a) une protéine de fusion comportant une protéine de sécrétion, un domaine d'ancrage à la membrane et une séquence de clivage de sécrétases,
b) une protéine comportant une activité sécrétase reconnaissant ladite séquence de clivage de ladite protéine de fusion et
c) au moins un gène rapporteur sous le contrôle d'un système d'activation de la transcription où ledit système d'activation de la transcription est régulé par la libération de ladite protéine de sécrétion de ladite protéine de fusion par ladite activité de sécrétase et la sécrétion subséquente
puis lesdites cellules sont ensuite cultivées dans des conditions appropriées
telles que ledit gène rapporteur permettant la détection et/ou la survie des cellules soit seulement exprimé ou réprimé d'une façon qui dépend de l'altération de l'activité de sécrétase due à ladite substance test.

2. Méthode pour l'isolation d'inhibiteur de sécrétases selon la revendication 1, dans laquelle une réduction ou une absence de libération de ladite protéine de sécrétion due à une activité sécrétase réduite/inhibée aboutit à l'expression d'au moins un desdits gènes rapporteurs permettant ainsi la détection et /ou la survie des cellules dans des conditions de cultures appropriées.

3. Méthode selon la revendication 1 ou 2, dans laquelle au moins un desdits gènes de rapporteurs est choisi parmi les gènes conférant une résistance aux antibiotiques, les gènes codant pour des molécules rapporteuses possédant une activité pouvant être détectée par des méthodes de colorimétrie ou de fluorescence et les gènes complétant les auxotrophies, de préférence un gène His 3.

4. Méthode pour l'identification d'agonistes d'activité sécrétase selon la revendication 1, dans laquelle la libération de ladite protéine de sécrétion due à une activité sécrétase augmentée aboutit à la réduction de l'expression d'au moins un desdits gènes rapporteurs permettant ainsi la détection et /ou la survie des cellules dans des conditions appropriées.

5. Méthode selon la revendication 4, dans laquelle au moins un desdits gènes rapporteurs est choisi parmi les gènes conférant la sensibilité à un produit chimique.

6. Méthode selon l'une quelconque des revendications précédentes, dans laquelle lesdites cellules comportent un second gène rapporteur choisi parmi le groupe consistant en :
a) des gènes codants pour des molécules rapporteuses possédant une activité pouvant être détectée par des méthodes de colorimétrie ou de fluorescence,
b) des gènes conférant une résistance aux antibiotiques et les gènes conférant une sensibilité à un produit chimique et
c) des gènes complétant les auxotrophies.

7. Méthode selon l'une quelconque des revendications précédentes, dans laquelle lesdites cellules sont des cellules de levures.

8. Méthode selon l'une quelconque des revendications précédentes, dans laquelle ladite protéine de sécrétion possède une activité enzymatique, de préférence une protéine possédant une activité invertase ou les fragments fonctionnels d'une protéine possédant une activité invertase.

9. Méthode selon l'une quelconque des revendications précédentes, dans laquelle ladite protéine de sécrétion est une invertase de levure ou les fragments fonctionnels d'une invertase de levure.

10. Méthode selon l'une quelconque des revendications précédentes, dans laquelle ladite séquence de clivage de la sécrétase est choisie dans le site β et le site α de la protéine précurseur humaine de l'amyloïde et dans le site S2 de la protéine Notch 1.

11. Méthode selon la revendication 10, dans laquelle les changements Lys595Asn et Met596Leu ont été introduits dans le site β.

12. Méthode selon l'une quelconque des revendications précédentes, dans laquelle ladite protéine de fusion comporte un signal de rétention au RE.

13. Méthode selon l'une quelconque des revendications précédentes, dans laquelle ladite protéine de fusion comporte les résidus acides aminés 1-532 de l'invertase de levure, les résidus acides aminés 590-695 de l'APP humaine et un signal de rétention au RE.

14. Méthode selon l'une quelconque des revendications précédentes, dans laquelle ladite protéine de fusion comporte les résidus acides aminés 1-532 de l'invertase de levure, les résidus acides aminés 1714-1876 de la protéine humaine Notch 1 et un signal de rétention au RE.

15. Méthode selon l'une quelconque des revendications précédentes, dans laquelle une construction d'acides nucléiques codant pour ladite protéine de fusion est intégrée au génome de ladite cellule hôte.

16. Méthode selon l'une quelconque des revendications précédentes, dans laquelle ladite protéine comportant une activité sécrétase reconnaissant ladite séquence de clivage de ladite protéine de fusion comprend aussi une séquence signal au RE.

17. Méthode selon l'une quelconque des revendications précédentes, dans laquelle ladite activité sécrétase est choisie parmi la β-sécrétase et l'α-sécrétase.

18. Méthode selon la revendication 16, dans laquelle ladite protéine comportant une activité β-sécrétase comprend également une séquence signal RE et les résidus acides aminés 616-695 de l'APP humaine.

19. Méthode selon l'une quelconque des revendications précédentes, dans laquelle ledit système d'activation de la transcription comporte au moins une partie du système de régulation du gène GAL de levure.

20. Méthode selon l'une quelconque des revendications précédentes, dans laquelle ledit gène rapporteur est placé sous le contrôle de la région de régulation GAL1-10 de levure.
